# EUROPÄISCHE PATENTSCHRIFT

(11) **EP 3 694 842 B1**
(45) Veröffentlichungstag und Bekanntmachung des Hinweises auf die Patenterteilung: **25.01.2023**
(21) Anmeldenummer: 18786724.7
(22) Anmeldetag: 10.10.2018
(51) Int. Cl.: C07D 307/68

(54) **VERFAHREN ZUR HERSTELLUNG VON FURAN-2,5-DICARBONSÄURE**
PROCESS FOR PREPARING 2,5-FURANDICARBOXYLIC ACID
PROCÉDÉ DE PRÉPARATION D'ACIDE 2,5-FURANEDICARBOXYLIQUE

(30) Priorität: 12.10.2017 DE 102017123797
(43) Veröffentlichungstag der Anmeldung: 19.08.2020
(73) Patentinhaber: Synphabase AG, 4133 Pratteln (CH)
(72) Erfinder: KASTL, Robert, 4410 Liestal (CH); KAUFMANN, Elias, 6004 Luzern (CH); BODENMÜLLER, Arthur, 4411 Seltisberg (CH); SEDELMEIER, Gottfried, 79227 Schallstadt (DE)
(74) Vertreter: Mertzlufft-Paufler, Cornelius
(86) Internationale Anmeldenummer: PCT/EP2018/077608
(87) Internationale Veröffentlichungsnummer: WO 2019/072920

(56) Entgegenhaltungen:
- WO-A1-2015/056270
- US-A1- 2007 232 815
- HANSEN THOMAS S ET AL: "Cu catalyzed oxidation of 5-hydroxymethylfurfural to 2,5-diformylfuran and 2,5-furandicarboxylic acid under benign reaction conditions", APPLIED CATALYSIS A: GENERAL, ELSEVIER, AMSTERDAM, NL, Bd. 456, 21. Februar 2013 (2013-02-21), Seiten 44-50, XP028582501, ISSN: 0926-860X, DOI: 10.1016/J.APCATA.2013.01.042

## Beschreibung

Die Erfindung betrifft ein neues Verfahren zur Herstellung von 2,5-Furandicarbonsäure, welches es ermöglicht, aus bei der Dehydratation von Hexosen oder hexosenhaltigen Materialien, jeweils aus Biomasse, wie vor allem Fructose, gewonnenem 5-Hydroxymethalfurfural (HMF) enthaltendem Rohmaterial in Form einer wässrigen Lösung oder Dispersion die 2,5-Furandicarbonsäure (FDCA) herzustellen.

FDCA ist ein Ausgangsmaterial für Polymere wie Polyethylenfuranoat (PEF), die in ihren Eigenschaften Polyethylenterephthalat (PET) vergleichbar sind, jedoch beispielsweise bessere Dichtigkeit gegenüber Gasdiffusion (z.B. bei Verwendung für Getränkeflaschen) und somit geringeres Oxidationsrisiko oder bei kohlensäurehaltigen Inhalten geringeren Verlust von CO₂ ermöglichen oder für synthetische Fasern aufgrund ihrer höheren Polarität gegenüber PET vorteilhafte Eigenschaften haben. Außerdem ist PEF beispielsweise leichter als PET.

BASF meldete im Jahre 2017 in https://www.basf.com/de/company/news-and-media/newsreleases/2017/06/p-17-246.html, dass für ein Industriekonsortium 25 Millionen Euro Zuschuss für PEF bereitgestellt wurden.

Kohlenhydrathaltige pflanzliche Biomasse kann als Quelle für aldehyd- und ketogruppenhaltige Verbindungen dienen, wobei diese über thermische, chemische oder enzymatische Verfahren zu Di-, Oligo- und Polysacchariden und dann weiter zu Monosacchariden wie Maltose, Xylose, Glucose oder insbesondere Fructose weiter zerlegt werden. Dabei sind zahlreiche andere Mischungsbestandteile wie Edukte, Haupt-, Zwischen-, Neben- oder Folgeprodukte Bestandteile der Reaktionsmischung, die als "Reaktionswasser" gewonnen werden kann. Aufwändige Reinigungsprozesse sind nötig; beispielsweise destillative Verfahren mit hohem Energieaufwand oder extraktive Aufarbeitung mit bedenklichen Lösungsmitteln und unerwünschten Nebenreaktionsmöglichkeiten für die Aldehyde und Ketone oder Verfahren unter Bindung an Aminogruppen tragende Adsorbentien wie in DE 110 2015 121 190 beschrieben können zur Gewinnung von 5-(Hydroxymethyl)furfural (HMF) eingesetzt werden.

Beispielsweise bei der thermischen Hydratation ("hydrothermal processing") insbesondere als Schritt nach oder bei einer hydrothermalen Karbonisierung (HTC) von (vor allem aus Biomasse gewonnenem) Hexosen (wie insbesondere Fructose) beinhaltendem Material entsteht HMF nach bisherigen Verfahren in Form eines Rohproduktes ("wässrige Lösung, Prozesswasser"), das eine Vielzahl von Nebenprodukten (wohl Oligomere, Kondensationsprodukte und dergleichen) enthält und oft von bräunlicher Farbe und vergleichsweise "klebrig" ist, siehe https://www.baselarea.swiss/baselarea-swiss/channels/ innovation-report/2016/08/muttenz-is-home-to-the-world-s-largest-plant-for-the-production-of-5-hmf-from-biomass.html.

Setzt man diese Produkte nach bisher bekannten Methoden unter Oxidation um, so entsteht ein schwer filtrierbares und aufarbeitbares Produkt, welches zudem hauptsächlich nur das einfach oxidierte Produkt, Furan-2,5-dicarbaldehyd, beinhaltet.

Die Oxidation von HMF aus Biomasse zu Furan-2,5-dicarbaldehyd mit dem Nitroxid TEMPO in Gegenwart sekundärer Oxidantien wie Natriumhypochlorit, Natriumbromit, Kalziumhypochlorit, Kupfer(I)chlorid und Sauerstoff, p-Toluolsulfonsäure, elektrochemisch erzeugtem radikalischen Brom oder Elektronen an Elektrodenoberflächen in einem biphasigen System (Wasser/Dichlormethan) beschreiben L. Cottier et al., J. Heterocycl. Chem. 1995, Seiten 927-930.

L. Hu et al., Renewable and Sustainable Energy Reviews 74, 230-257 (2017) beschreiben die Herstellung von Furandicarbonsäuremethylester ohne Herstellung der freien Säure aus HMF über Gold/Ceramid-Katalysatoren. Weitere Schriften wie US 2008/0103318 A1, US 9,617,234 B1 und WO 2017/106591 A1 beschreiben ebenfalls die Gewinnung von FDCA aus durch Dehydratation von Zuckern gewonnenem HMF mit schwermetallischen Katalysatoren wie trägergestützten Gold-, Platin- oder Palladiumkatalysatoren mit molekularem Sauerstoff aus Luft. Nachteilig ist, dass derartige Katalysatoren bei den aus der Hydratation von Zuckern resultierenden sehr rohen Mischungen schnell verklebt werden und verschmutzen und so einerseits als Abfall anfallen, andererseits rasch ausgetauscht und erneuert werden müssen.

WO 2016/191682 beschreibt die Gewinnung von Furan-2.5-dicarbonsäuredichlorid (FDCC) aus 5-(Chlormethyl)furfural, das mittels eines Wismuth-haltigen Katalysators zu Furan-2,5-dicarbaldehyd (DFF) umgewandelt wird, welches dann in einer zweiten Reaktion mittels tert.-Butyl-hypochlorit unter wasserfreien Bedingungen in FDCC umgewandelt wird.

Thomas S. Hansen et al., Applied Catalysis A: General, Elsevier, Amsterdam (NL), Vol. 456, 21. Feb. 2013, p. 44-50 beschreibt die kupferkatalysierte Oxidation von 5-Hydroxymethylfurfural zu 2,5-Furandicarbonsäure mit Cu(I) und als stöchiometrischem Oxidationsmittel t-Butylperoxid (t-BuOOH) oder alternativ die Oxidation zu 2,5-Dimethylfuran ((also nicht zu 2,5-Furandicarbonsäure) mit Sauerstoff in Gegenwart von TEMPO und Cu(I). Als Edukt wird reines 5-Hydroxymethylfurfural (">99 %") in den Beispielen eingesetzt.

WO 2015/056270 A1 beschreibt eine Reaktionsfolge, bei der zunächst 5-Hydroxymethylfurfural (in den Beispielen offenbar eine reine Form) durch Acylierung mittels eines reaktiven Säurederivates (Säurechlorid oder Säureanhydrid) in ein 5-Acyloxymethylfurfural umgewandelt wird, der dann in einem ersten Oxidationsschritt mittels eines oxidierenden Agens in die entsprechende 5-Acyloxymethylfurancarbonsäure umgewandelt wird, welche dann in einem zweiten Oxidationsschritt zu Furandicarbonsäure umgesetzt wird. Im ersten Oxidationsschritt wird in den Beispielen als Oxidationsmittel Natriumchlorit und Wasserstoffperoxid verwendet, im zweiten Oxidationsschritt wird Salpetersäure oder Kaliumpermanganat unter basischen Bedingungen als Oxidationsmittel verwendet. Eine Reihe weiterer Oxidationsmittel wird erwähnt. Hier sind also eine Vielzahl von Reaktionsschritten erforderlich, und die Acylierung erfordert Abwesenheit von Wasser im Ausgangsmaterial (reines 5-Hydroxymethylfurfural).

US 2007/232815 A1 nennt eine Methode zur Herstellung von Furan-2,5-dicarbonsäure unter Verwendung von Metallpermanganaten in basischem Medium. Als Edukte werden reine Substanzen, beispielsweise reines 5-Hydroxymethylfuran (99 % Reinheit), verwendet.

Vor diesem Hintergrund bestand die Aufgabe, ein Verfahren zur Herstellung von FDCA bereitzustellen, welches ökologisch vorteilhaft ist und vor allem einfach und für die Massenproduktion von FDCA geeignet ist.

Dies ermöglicht insbesondere ein nachhaltiges Produzieren auf Basis biologischer Ausgangsmaterialien von PEF und anderen Produkten, die aus FDCA erhältlich sind und ermöglicht somit, den Verbrauch von Edukten aus fossilen Kohlenstoffverbindungen zu ersetzen.

Vorteilhaft wäre auch ein Verzicht auf edel- oder schwermetallhaltige Katalysatoren.

Es wurde nun überraschend gefunden, dass bei geeigneten Reaktionsbedingungen, insbesondere unter basischen Bedingungen, in Gegenwart eines Oxidationsmittels in einer Eintopfreaktion in einem chargenweisen Verfahren (Batch-Processing) oder einem Verfahren nach dem Fließprinzip (Continuous Flow Processing) die Oxidation von HMF zu FDCA in einer wässrigen Reaktionsmischung wie in Anspruch 1 beschrieben möglich ist.

Dies ermöglicht gleichzeitig die Oxidation störender Nebenprodukte, so dass auch aus bräunlich verfärbten Edukten nahezu farblose Lösungen hergestellt werden können.

Das nachfolgende Schema veranschaulicht die wesentlichen Aspekte der vorliegenden Erfindung (ohne sie zu beschränken):

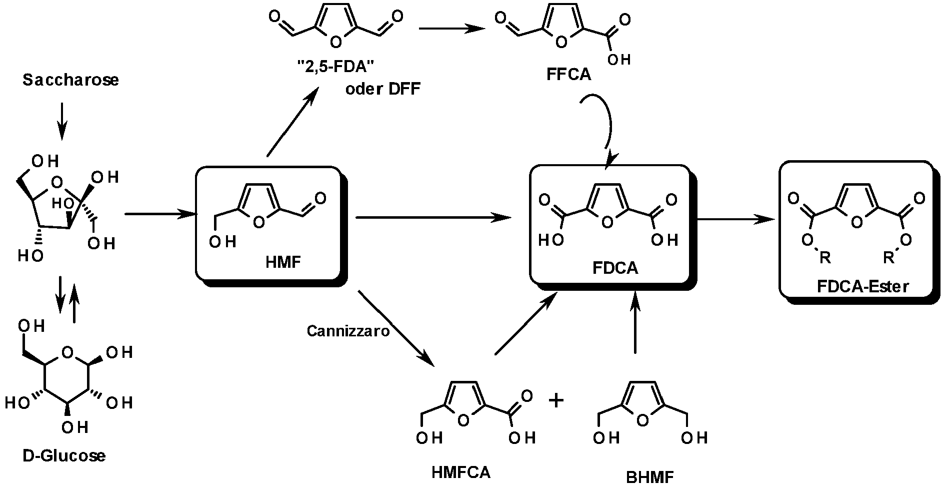

Die Erfindung betrifft daher in einer ersten Ausführungsform ein Verfahren zur Herstellung von Furan-2,5-dicarbonsäure, beinhaltend die Oxidation von 5-Hydroxymethylfurfural in einer flüssigen Reaktionsmischung in einem Eintopfverfahren oder in einem Continuous Flow-Verfahren; wobei als Oxidationsmittel ein Metallhypohalogenitsalz eingesetzt wird und es sich bei dem Ausgangsmaterial 5-Hydroxymethylfurfural um ein bei der hydrothermalen Karbonisierung von Hexosen oder hexosehaltigen Materialien aus Biomasse gewonnenes Rohmaterial in Form einer wässrigen Lösung oder Dispersion handelt.

Die Erfindung betrifft in einer zweiten Ausführungsform auch die Verwendung des besagten Oxidationsmittels zur Herstellung von Furan-2,5-dicarbonsäure aus 5-Hydroxymethylfurfural in Form des vorstehend erwähnten Rohmaterials in einer flüssigen Reaktionsmischung in einem Eintopfverfahren, insbesondere in einem Verfahren wie vor- und nachstehend definiert.

Die vor- und nachstehend verwendeten allgemeineren Ausdrücke für Merkmale haben vorzugsweise die nachstehend aufgeführten spezifischeren Definitionen, wobei in jeder Ausführungsform der Erfindung ein oder mehrere oder alle allgemeineren Ausdrücke durch die nachstehend genannten spezifischeren Ausdrücke ersetzt werden können, was zu bevorzugten Ausführungsformen der Erfindung führt.

HMF in Mischungen aus biologischen Prozessen selbst ist das zu oxidierende Edukt.

Eine flüssige Reaktionsmischung ist ein wässriges System mit oder ohne mit Wasser mischbaren (einphasige Reaktionsführung) oder insbesondere mit Wasser nicht mischbaren organischen Lösungsmitteln (Phasentransferreaktion).

Bei einem Eintopfverfahren, hier als Batch-Verfahren, geschieht die Prozessierung direkt, es wird in einem Eintopfverfahren das Endprodukt FDCA hergestellt. Bevorzugte Reaktionsbedingungen finden sich unten.

Bei einem Verfahren nach dem Fließprinzip (Continuous Flow Processing) geschieht die Umsetzung vom Edukt zum Produkt kontinuierlich unter Bewegung der Reaktionsmischung von einem Startpunkt mit kontinuierlicher Edukt-Zugabe und fortlaufender Reaktion während der Bewegung bis zu einem Endpunkt mit kontinuierlicher Produktentnahme ohne Unterbrechungen. In diesem Fall kann das FDCA in einem kontinuierlichen Prozess aus dem Edukt (z.B. Rohprodukt der HTC) gewonnen werden.

Als Oxidationsmittel dient ein Metallhypohalogenit. Als Metall (in kationischer Form) sind insbesondere Alkali-, Erdalkali- oder andere Metalle (in Form ihrer Salze) zu nennen. Besonders bevorzugt sind KOCl, LiOCl, NaOCl, Ca(OCl)₂ oder eine Mischung von zwei oder mehr dieser Hypochlorite. Beispielsweise kann sehr vorteilhaft und einfach Natriumhypochlorit und/oder Kaliumhypochlorit in Form von Javel-Wasser (Eau de Javel) (Lösung von Natriumhypochlorit und/oder Kaliumhypochlorit, oft zusammen mit Natriumchlorid oder Kaliumchlorid) verwendet werden. Auch Mischungen von zwei oder mehr derartigen Oxidationsmitteln sind möglich.

Wo "ferner" verwendet wird, bedeutet dies, dass Merkmale oder Materialien, die ohne dieses Attribut angegeben sind, gegenüber den nach "ferner" genannten bevorzugt sind.

Das Edukt, 5-Hydroxymethylfurfural, ist ein Rohmaterial, das bei der Dehydratisierung von Hexosen oder hexosehaltigen Materialien aus Biomasse, wie Glucose oder insbesondere Fructose, gewonnen ist (siehe z.B. obiges vereinfachtes Reaktionsschema), wobei das hexosehaltige Material insbesondere als "Neben"produkt einer Hydrothermalen Carbonisierung erhalten sein kann. Es liegt dann in wässriger Lösung oder Dispersion (Emulsion oder Suspension) vor (nachfolgend beide als "Lösung" oder als (ggf. wässriges) Rohmaterial bezeichnet). Oft ist es gefärbt und enthält eine Reihe von Nebenprodukten, die es "klebrig" und viskos machen können.

Die Oxidation wird vorzugsweise unter basischen Bedingungen geführt, beispielsweise eingestellt durch Basen wie basische Alkali- oder Erdalkalimetallsalze, wie die entsprechenden Carbonate oder Phosphate, oder durch Alkalimetallhydroxide, wie Natrium- oder Kaliumhydroxid. Vorzugsweise bedeutet "basische Bedingungen", dass die Reaktion in einem pH-Bereich von größer als 9, vorzugsweise von 9,5 bis 13, z.B. von 9.8 bis 12,5, stattfindet. Der pH kann vorzugsweise durch kontinuierliche Zugabe der entsprechenden Base konstant gehalten werden.

Die Oxidation erfolgt vorzugsweise in Gegenwart von einem Nitroxylradikal als Katalysator, vorzugsweise von einem Tetramethylpiperidinoxid, wie 2,2,6,6-Tetramethylpiperidin-1-oxyl (TEMPO), 2,2,6,6-Tetramethylpiperidin-1-oxy-4-ol ("4-OH-TEMPO" oder "TEMPOL"), 1-Oxyl-2,2,6,6-tetramethylpiperidin-4-on (TEMPON), 1-Oxyl-2,2,6,6-tetramethyl-4-carboxyl-piperidin (4-Carboxy-TEMPO), 1-Oxyl-2,2,5,5-tetramethylpyrrolidin, 1-Oxyl-2,2,5,5-tetramethyl-3-carboxylpyrrolidin (3-Carboxy-PROXYL), 4-Benzoyloxy-2,2,6,6-tetramethylpiperidin-N-Oxyl, 4-methoxy-2,2,6,6-tetramethylpiperidin-N-Oxyl, 4-oxo-2,2,6,6-tetramethylpipieridin-N-Oxyl, ferner Aluminium-N-nitrosophenylhydroxylamin oder Diethylhydroxylamin. Das Nitroxylradikal kann in freier Form oder (beispielsweise über reaktive Gruppen wie Hydroxy- oder Carboxygruppen, an Trägermaterialien, insbesondere polymere Materialien, gebunden vorliegen, was insbesondere die Anwendung in einem Continuous-Flow-Verfahren erleichtert und eine Abtrennung eines gelösten Nitroxylradikals vermeiden hilft bzw. die Rückgewinnung des trägergebundenen Nitroxylradikals sehr vereinfacht. Ein Beispiel für dearartige trägergebundene Nitroxylradikale sind über Aza-derivatisierung reaktivierte Epoxygruppem aufweisende Harze, an die Nitroxylradikale mit durch Propargylbromid funktionalisierte Hydoxygruppen kovalent gebunden werden, wie z.B. in A. Bogdan und D.T.McQuade, Beilstein J. Organ. Chem. 5(17) 2008, beschrieben (basierend auf AMBERZYME ^{®} Oxiran, azidmodifiziert, und 4-Hydroxy-TEMPO); auch Varianten, in denen über eine Carboxygruppe z.B. an Aminogruppen tragende Trägerpolymere (beispielsweise durch Reaktion mit Kondensationsreagentien wie aus der Peptidchemie bekannt oder durch Reaktion der entsprechenden Carbonsäurehalogenide, wie - Chloride, mit Carboxylgruppen tragenden Nitroxylradikalen, wie 4-Carboxy-TEMPO oder 3-Carboxy-PROXYL), sind alternativ einsetzbar. Auch Mischungen von zwei oder mehr freien oder trägergebundenen Nitroxylradikalen sind möglich.

Die erfindungsgemäße Oxidation wird insbesondere mit einem molaren Überschuss des Oxidationsmittels gegenüber 5-Hydroxymethylfurfural durchgeführt, vorzugsweise mit dem 4- oder mehr-, insbesondere dem 4,5- bis 7-fachen molaren Überschuss gegenüber dem 5-Hydroxymethylfurfural.

Insbesondere im Batchverfahren, aber auch im Continuous-Flow-Verfahren, wird die Reaktion einphasig oder vorzugsweise unter Phasentransferbedingungen (d.h. bei Vorliegen einer wässrigen und einer mit der wässrigen Phase nicht mischbaren organischen Phase), insbesondere in Gegenwart eines Phasentransferkatalysators, durchgeführt.

Als Phasentransferkatalysatoren kommen dabei insbesondere quartäre Ammoniumsalze (vor allem für Anionentransfer, wie den Transfer von FDCA in anionische Form in das wässrige Medium gebräuchlich) oder ferner Kronenether (eher für den Transfer von Kationen gebräuchlich) zum Einsatz. Beispiele sind Tetraalkylammonium- wie Tetrabutylammonium- oder Dodecyltrimethylammonium-Sulfat oder -halogenide, wie -bromid, -chlorid oder -sulfat.

Für die mit Wasser nicht mischbare organische Phase werden apolare oder schwach polare, hydrophobe Lösungsmittel oder Mischungen solcher Lösungsmittel, eingesetzt.

Unter Lösungsmitteln sind dabei in erster Linie wasserunlösliche Lösungsmittel wie gesättigte Kohlenwasserstoffe, insbesondere lineare, verzweigte oder cyclische Alkane wie Pentan, Hexan, Octan, Cyclohexan, aromatische Kohlenwasserstoffe, wie Benzol oder Alkylbenzole, wie Mono-, Di-, Tri-, Tetra-, Penta- oder Hexa-C₁-C₄Alkylbenzole, wie Toluol, Xylen, Cumen, Ethylbenzol, oder dergleichen, oder für einphasige Systeme auch wassermischbare Lösungsmittel wie tert.-Butylakohol, Alkylcyanide, wie Acetonitril oder Propionitril, ionische Flüssigkeiten (Salze in flüssiger Form), z.B. Salze von Imidazolium, Pyrrolidinium, Pyridinium, Guanidinium, Uronium, Thiouronium, Piperidinium, Ammonium und Phosphonium als Anion und Halogenid, wie Chlorid oder Fluorid oder Bromid, Tetrafluorborat, Trifluoracetat, Triflat, Hexafluorphosphat, Phosphinate oder Tosylat als Anion, wie 1-Butyl-3-methylimidazolium-hexafluorphosphat, oder (insbesondere aus Biomasse gewinnbare) beta-, gamma- oder delta-Lactone, Hydrofurane, Hydropyrane, Dioxan oder Kombinationen davon, wie gamma-Valerolacton, und dergleichen, oder ferner Dialkylformamide, wie Dimethylformamid, (wasserunlösliche) halogenierte Kohlenwasserstoffe, z.B. teilweise oder perhalogenierte Kohlenwasserstoffe, wie Dichlormethan, Chloroform, Tetrachlormethan, Hexachlorethan, Hexafluorethan oder dergleichen, zu verstehen bzw. werden eingesetzt. Mischungen von zwei oder mehr der genannten Lösungsmittel sind auch möglich.

Für einphasige Reaktionen sind auch wasserlösliche Lösungsmittel, wie oben genannt, möglich. Diese können, soweit sie nicht die Ausbildung von zwei Phasen stören, auch bei zweiphasigen Systemen dem Reaktionsgemisch zugegeben sein.

Als Metallhalogenid werden vorzugsweise Alkali- oder Erdalkalimetallhalogenide, insbesondere -bromide oder Chloride, eingesetzt. Bevorzugt sind Lithiumbromid, Natriumbromid oder Kaliumbromid. Diese Verbindungen können während der Reaktion in die entsprechenden Hypohalogenite, insbesondere Hypobromite, umgewandelt werden und so die Reaktion beschleunigen.

Die Reaktion kann auch dergestalt geführt werden, dass zunächst oder als Bestandteil der erfindungsgemäßen Oxidation eine Disproportionierung zu 5-Hydroxymethyl-furan-2-carbonsäure und 2,5-Dihydroxyxmethylfuran unter basischen Bedingungen stattfindet (insbesondere unter Bedingungen der Cannizzaro-Reaktion, insbesondere in Gegenwart von Alkalimetallhydroxid oder durch Mischung mit Alkalimetallhydrid), das heißt, das HMF selbst dient gleichzeitig als Oxidationsmittel und als Edukt für 5-Hydroxymethyl-furan-2-carbonsäure als Vorstufe von FDCA, die dann, gewünschtenfalls nach Aufreinigung, insbesondere zur Abtrennung von 2,5-Dihydroxymethylfuran, weiter unter Herstellung von FDCA unter den erfindungsgemäßen Oxidationsbedingungen umgesetzt werden kann bzw. wird. Diese Art der Reaktionsführung ist dann besonders bevorzugt, wenn auch Dihydroxymethylfuran als Produkt gewünscht wird. Dies kann beispielsweise selbst als Diol für die PEF-analoge Herstellung entsprechender Kunststoffe verwendet werden, oder für andere synthetische Zwecke.

Es ist auch möglich, die Furan-2,5-dicarbonsäure (vorzugsweise unter wasserfreien Bedingungen) weiter zu einem Derivat wie einem Ester (mit Alkoholen, wie Ethanol oder Methanol, vorzugsweise in Gegenwart eines Sulfonyl- oder besser Thionylhalogenides, wie -chlorides, oder eines organischen Carbonsäure-Halogenides, wie -chlorides, und jeweils des betreffenden Alkohols, wie Methanol oder Ethanol) oder einem reaktiven Säurederivat (wie dem Säurehalogenid, z.B. -chlorid), oder einem reaktiven Ester, wie 4-Nitrophenylester (mit dem entsprechenden Phenol), oder einem Anhydrid (Anhydrid aus zwei oder mehr FDCA-Molekülen selbst oder gemischter Anhydrid mit einer anderen Säure, wie Essigsäure oder Propionsäure), an einer oder beiden Carboxylgruppen umzusetzen, um so weitere nützliche Produkte und Verbindungen für weitere erwünschte Umsetzungen zu erhalten. Dies kann auch genutzt werden, um nach Aufreinigung und Hydrolyse besonders reine FDCA zu gewinnen, und/oder die Ester, wie Dimethyl- oder Diethylester von FDCA, können direkt (vorzugsweise nach Aufreinigung) mit Polyolen oder Diolen, wie oben genannt, zu PEF umgesetzt werden.

Die vorstehend beschriebenen erfindungsgemäßen Verfahren zur Herstellung von FDCA beinhalten in einer bevorzugten Ausführungsform der Erfindung auch die Herstellung des Ausgangsmaterials 5-Hydroxymethylfurfural durch Dehydratisierung von Hexosen oder insbesondere hexosehaltigen Materialien aus Biomasse, wie aus Glucose oder insbesondere Fructose aus Biomasse.

Die Dehydratisierung kann dabei nach einem der in US 2008/0103318 A1, US 9,617,234 B1, WO 2017/106591 beschriebenen Verfahren oder wie in FR 2723945 beschrieben erfolgen, insbesondere im Anschluss an eine Hydrothermale Carbonisierung (HTC), in Gegenwart einer Säure, wie Salpetersäure, in wässrigem Medium, vorzugsweise bei erhöhten Temperaturen, wie 150 bis 150 °C, wobei ein (wässriges) Rohprodukt (Rohmaterial) wie oben erwähnt erhalten wird.

Hexose-, wie fruktosehaltiges, Edukt wird dabei in einer bevorzugten Variante gemeinsam mit Wasser und dem Katalysator erhitzt. Unter Druck reagiert Fruktose unter Wasserabspaltung. Das Prozesswasser wird optional anschließend filtriert. Wenn man das Wasser verdampfen würde, würde man beispielsweise den Feststoff HMF zusammen mit noch unreagierter Fructose und 20% anderen Materialien (z.B. Kohle, Essigsäure, Ameisensäure, Humine, Lävulinsäure etc.) erhalten. Jedoch behält man vorzugsweise HMF in flüssiger Form für den nächsten Schritt der FDCA Gewinnung (erfindungsgemäße Oxidation) als wässrige Lösung ("Prozesswasser", "Rohprodukt").

Das Verfahren zur Herstellung von FDCA kann in einer weiteren bevorzugten Ausführungsform der Erfindung ergänzt werden (vorzugsweise nach Isolierung von FDCA, beispielsweise durch Chromatographie, Ausschütteln oder Kristallisation, und gewünschtenfalls Umkristallisation) durch die anschließende Umsetzung mit einem Diol, wie einem Di- oder Polyol, wie einem Zuckeralkohol, Isosorbid, 2,5-Dihydroxyxmethylfuran oder insbesondere Ethylenglykol) unter Bildung von Polyesterharzen, die prinzipiell PET ähneln.

Die Kristallisation und Umkristallisation von FDCA nach deren Erhalten durch die erfindungsgemäße Oxidation stellt dabei ebenfalls eine Ausführungsform der Erfindung dar.

Bei einem Batch-Verfahren geschieht die Prozessierung von Materialien in Chargen (separaten Ansätzen) über jeden Schritt des gewünschten Prozesses (hier alle Oxidationsschritte), die Prozessierung nachfolgender Chargen muss warten bis eine komplette Umsetzung beendet ist.

Bei einem Verfahren nach dem Fließprinzip (Continuous Flow Processing) geschieht die Umsetzung vom Edukt zum Produkt kontinuierlich unter Bewegung der Reaktionsmischung von einem räumlichen Startpunkt mit kontinuierlicher Eduktzugabe und fortlaufender Reaktion während der Bewegung bis zu einem (räumlich getrennten) Endpunkt mit kontinuierlicher Produktentnahme ohne Unterbrechungen.

Die Erfindung betrifft insbesondere auch die jeweils in den Ansprüchen genannten Ausführungsformen, weshalb die Ansprüche hier auch als zur Beschreibung gehörig anzusehen sind.

### Es zeigt:

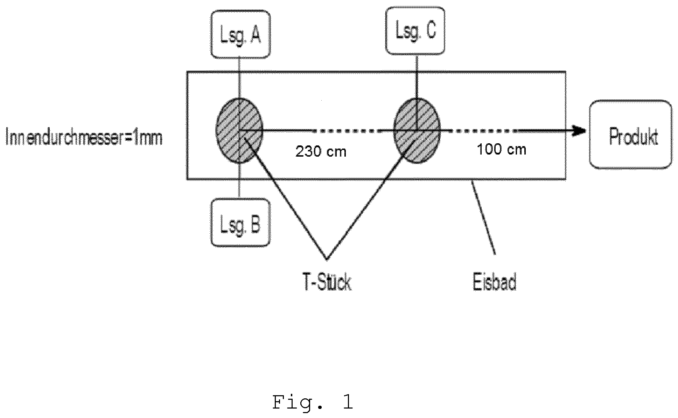

Figur 1: Schematische Darstellung einer Vorrichtung zur 5-Hydroxymethylfurfural (HMF) Oxidation nach dem Fließprinzip (im Continuous Flow)

Die nachfolgenden Beispiele dienen der Illustration der Erfindung, stellen jedoch auch bevorzugte Ausführungsformen der Erfindung dar.

### Beispiel 1: 5-Hydroxymethylfurfural (HMF) Oxidation mit TEMPO/NaOCl zu 2,5-Furandicarbonsäure (FDCA) im Eintopfverfahren (chargenweises (= Batch-) Verfahren, kg-Maßstab)

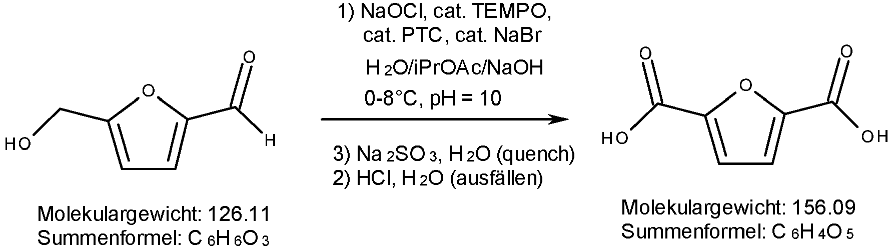

iPrOAc ist Isopropylacetat, PTC steht für Phase Transfer Catalyst = Phasentransferkatalysator (Tetrabutylammoniumhydrogensulfat)

Es wird angenommen, ohne dass dies als bindende Beschreibung anzusehen ist, dass diese Reaktion über folgende Zwischenprodukte stattfindet:

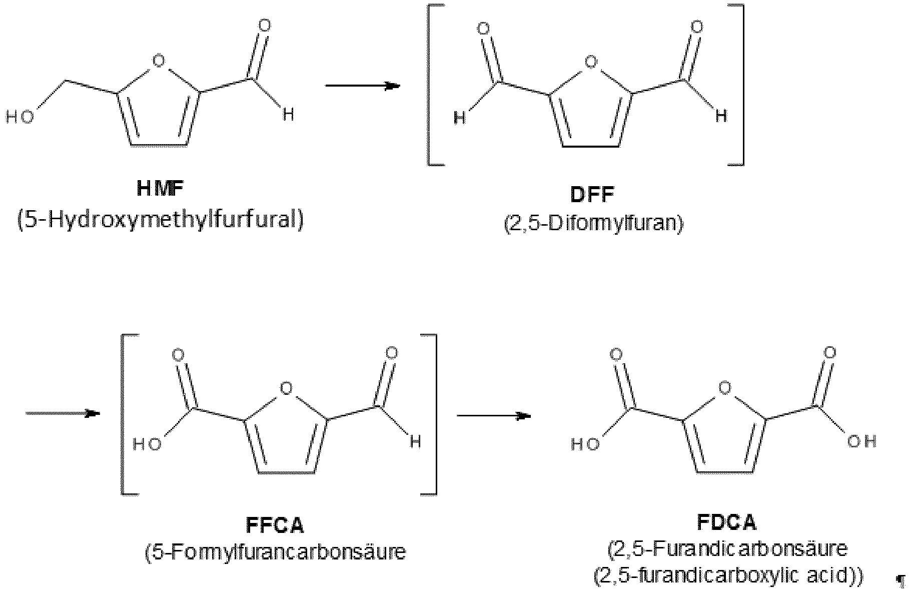

Im 160 L-Reaktor wurden wässrige HMF-Lsg. (18%ig, 17.0 L, 25.0 mol, 1.0 eq; Rohprodukt nach Hydratation Hexosehaltiger Biomasse) vorgelegt und bei 5°C mit Tetrabutylammoniumhydrogensulfat (52.0 g, 0.150 mol, 0.6 mol%) als Phasentransferkatalysator, Natriumbromid (2.06 kg, 20 mol, 0.8 eq), TEMPO (200 g, 1.25 mol, 0.05 eq) und Isopropylacetat (8 L) versetzt. Unter kräftigem Rühren wurde bei 0-8°C Javelwasser (auf NaOCl-Basis; 13-14%, 137.5 mol, 5.5 eq) langsam zugetropft während 6 h. Der pH-Wert des Reaktionsgemisches (RG) wurde durch kontinuierliche Zugabe von Natronlauge 30% bei pH = 10-11 gehalten. Nachdem Interner Prozesskontrolle (IPC) (1H-NMR in D₂O der wässrigen Phase des RG) keine Intermediate (DFF, FFCA) mehr zeigte, wurde die organische Phase des RG abgetrennt und die wässrige Phase mit Isopropylacetat (2 x 10 L) extrahiert bei < 10°C. Die organischen Phasen wurden verworfen. Die wässrige Phase wurde zu einer kalten (< 10°C) wässrigen Lösung aus Natriumsulfit (9.5 kg, 75.0 mol, 3.0 eq) in 60 L deionisiertem Wasser gegeben und 1 h bei 5°C ausgerührt. Die Lösung wurde anschließend bei 0-5°C langsam mit Salzsäure 32% versetzt bis pH < 1. Die resultierende Suspension wurde filtriert und der Nutschkuchen (FDCA) wurde mit kaltem deionisiertem Wasser (1 × 10 L, 2 × 5 L) gewaschen. Die feuchte FDCA wurde in deionisiertem Wasser (60 L) suspendiert, mittels Natronlauge 30% in Lösung gebraucht und durch Zugabe von Salzsäure 32% wieder ausgefällt bei pH < 1 und 0-5°C. Nach 1 h Ausrühren bei 0-5°C wurde abfiltriert und mit kaltem deionisiertem Wasser (1 × 10 L, 2 × 5 L) nachgewaschen. Die feuchte FDCA wurde im Vakuum bei 60°C getrocknet. Es wurden 3.1 kg (80%) FDCA als weißes Pulver erhalten.

HPLC @220 nm 98.9% Reinheit, NMR mit int. Standard 98.4% Gehalt. Schmelzpunkt: ab 330 ° C unter Zersetzung.

¹H-NMR (400 MHz, DMSO-d₆) : *δ* = 13.63 (br s, 2H), 7.30 (s, 2H) .

MS (ESI): m/z = 156.9 [*M*+H]⁺

### Beispiel 2: 5-Hydroxymethylfurfural (HMF) Oxidation mit TEMPO/NaOCl zu 2,5-Furandicarbonsäure (FDCA) nach dem Fließprinzip (im Continuous Flow)

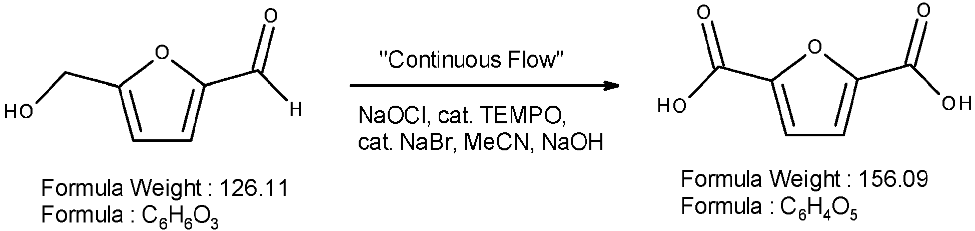

Die entsprechende Vorrichtung ist in Fig. 1 gezeigt (Durchflussreaktor). Es handelt sich um ein Röhrchen aus Teflon mit einem Innendurchmesser von 1 mm.

### Drei Lösungen wurden vorbereitet:

Lösung A): 6.35 g (5.0 mmol) 5-Hydroxymethylfurfural, 4.22 g (4.1 mmol) NaBr wurden in 29 g deionisiertem Wasser gelöst und mit einer Lösung aus 0.80 g (0.5 mmol) 2,2,6,6-Tetramethylpiperidin-1-oxyl in 20 mL (15.4 g) Acetonitril vermischt. Die hergestellte Lösung erreichte eine Konzentration von 0.90 mol/L (HMF/Lösung).

Lösung B): kommerziell erhältliche NaOCl Lösung (13-14%) mit einer Konzentration von 2.26 mol/L (NaOCl/Lösung)

Lösung C): 3.0g (1.5 mmol) NaOH gelöst in 55 mL (68.8g) 13-14% NaOCl Lösung mit einer Endkonzentration von 1.36 mol/L (NaOH/Lösung).

Mittels Spritzenpumpen wurden die Lösung A mit einer Flussrate von 0.111 mL/min, die Lösung B mit einer Flussrate von 0.139 mL/min und die Lösung C mit einer Flussrate von 0.120 mL/min links in den Durchflussreaktor nach Fig. 1 gepumpt. Es wurde zu Beginn das dreifache Totvolumen verworfen und anschließend die Reaktionslösung auf der rechten Seite für 70 Minuten gesammelt (NMR zeigt sauberen und vollständigen Umsatz zum Produkt). Die Reaktionslösung wurde anschließend aufgearbeitet wie im Batch-Versuch beschrieben und das gereinigte FDCA erhalten.

### Beispiel 3: 2,5-Furandicarbonsäure (FDCA) Veresterung zu 2,5-Furandicarbonsäure-dimethylester (FDME) mit SOCl₂ Batch kg-Maßstab

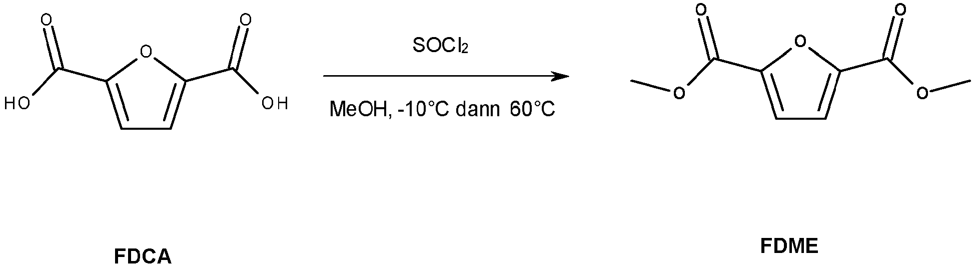

Im 160 L Reaktor wurden FDCA (9.58 kg, 60.15 mol, 1.0 eq) in 62 L Methanol vorgelegt. Bei -10 bis -5°C wurde langsam Thionylchlorid (10.6 L, 144.4 mol, 2.4 eq) zugetropft. Nach vollständiger Zugabe wurde das Reaktionsgemisch auf Rückfluss erwärmt. IPC (HPLC) zeigte nach 2 h 97% FDME, 1% Monoester und keine FDCA mehr. Das Reaktionsgemisch wurde auf -5°C abgekühlt und über Nacht bei -5°C ausgerührt. Anschliessend wurde abfiltriert und der Nutschkuchen (FDME) mit kaltem Methanol (2 x 5 L) nachgewaschen. Der noch feuchte FDME wurde in 100 L Aceton gelöst bei 25°C, mit Aktivkohle (400 g) versetzt und 30 min ausgerührt bei 25°C. Anschliessend wurde über Hyflo (Filterhilfsmittel, Kieselsäure) abfiltriert und das Filtrat wurde unter Vakuum bei 40°C aufkonzentriert auf 40-50 L. Die resultierende Suspension wurde abgekühlt auf -5°C und über Nacht bei -5°C ausgerührt. Anschliessend wurde abfiltriert und der Nutschkuchen mit kaltem Methanol (1 × 10 L und 1 × 5 L) nachgewaschen. Das Produkt wurde unter Vakuum bei 40°C getrocknet. Es wurden 9.22 kg (83%) FDME als weisses kristallines Pulver erhalten.

HPLC @220 nm 99.7% Reinheit, NMR mit int. Standard 99% Gehalt. ¹H-NMR (400 MHz, DMSO-d₆): *δ* = 7.44 (s, 2H), 3.87 (s, 6H). MS (ESI): m/z = 185.0 [*M*+H]⁺

### Beispiel 4: 2,5-Furandicarbonsäure (FDCA) Umkristallisation Gramm-Maßstab

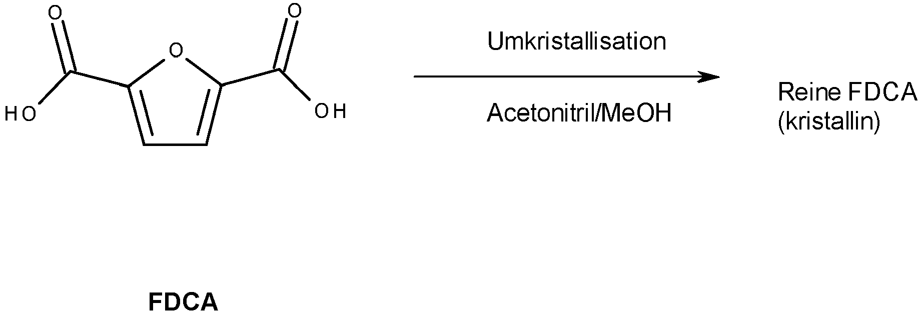

Im 2.5 L Sulfierkolben wurden FDCA (97% Reinheit, 60.0 g, 0.373 mol) in Acetonitril (960 mL) und Methanol (600 mL) vorgelegt und auf Rückfluss (76°C) erwärmt. Nach 30 min bei Rückfluss wurde die gelbe Lösung in 2 h auf 5°C abgekühlt. Bei 61°C begann FDCA auszukristallisieren. Die Suspension wurde bei 5°C abfiltriert und der Nutschkuchen mit kaltem Acetonitril (2 x 60 mL) nachgewaschen. Das Produkt wurde unter Vakuum bei 60°C getrocknet. Es wurden 43.7 g (75%) FDCA als weisses Pulver erhalten. HPLC @220 nm 99.6% Reinheit, NMR mit int. Standard 99% Gehalt.

¹H-NMR (400 MHz, DMSO-d₆) : *δ* = 13.63 (br s, 2H), 7.30 (s, 2H) . MS (ESI): m/z = 156.9 [*M*+H]⁺

## Patentansprüche

1. Verfahren zur Herstellung von Furan-2,5-dicarbonsäure, beinhaltend die Oxidation von 5-Hydroxymethylfurfural in einer flüssigen Reaktionsmischung in einem Eintopfverfahren oder in einem Continuous Flow-Verfahren; wobei als Oxidationsmittel ein Metallhypohalogenitsalz eingesetzt wird und es sich bei dem Ausgangsmaterial 5-Hydroxymethylfurfural um ein bei der hydrothermalen Karbonisierung von Hexosen oder hexosehaltigen Materialien aus Biomasse gewonnenes Rohmaterial in Form einer wässrigen Lösung oder Dispersion handelt.

2. Verfahren nach Anspruch 1, **dadurch gekennzeichnet, dass** es sich bei dem hexosehaltigen Material um Fructose handelt.

3. Verfahren nach Anspruch 1 oder 2, **dadurch gekennzeichnet, dass** die Reaktion unter Phasentransferbedingungen durchgeführt wird.

4. Verfahren nach einem der vorstehenden Ansprüche, **dadurch gekennzeichnet, dass** die Reaktion unter Phasentransferbedingungen in Gegenwart eines Phasentransferkatalysators durchgeführt wird, vorzugsweise in Gegenwart von Tetraalkylammonium- wie Tetrabutylammonium- oder Dodecyltrimethylammonium-Sulfat oder -halogenide, wie - bromid, -chlorid oder -sulfat.

5. Verfahren nach einem der vorstehenden Ansprüche, **dadurch gekennzeichnet, dass** die Reaktion unter basischen Bedingungen geführt wird, vorzugsweise eingestellt durch Basen, z.B. basische Alkali- oder Erdalkalimetallsalze, wie die entsprechenden Carbonate oder Phosphate, oder durch Alkalimetallhydroxide, wie Natrium- oder Kaliumhydroxid;wobei "unter basischen Bedingungen" insbesondere bedeutet, dass die Reaktion in einem pH-Bereich von größer als 9, vorzugsweise von 9,5 bis 13, z.B. von 9.8 bis 12,5, stattfindet, wobei der pH-Wert vorzugsweise durch kontinuierliche Zugabe der entsprechenden Base konstant gehalten wird.

6. Verfahren nach einem der vorstehenden Ansprüche, wobei die Oxidation mit KOCl, LiOCl, NaOCl, Ca(OCl)₂ oder einer Mischung von zwei oder mehr dieser Hypochlorite als Oxidationsmittel durchgeführt wird.

7. Verfahren nach einem der vorstehenden Ansprüche, wobei die Oxidation in Gegenwart von einem Nitroxylradikal als Katalysator durchgeführt wird, vorzugsweise von einem Tetramethylpiperidinoxid, wie 2,2,6,6-Tetramethylpiperidin-1-oxyl (TEMPO), 2,2,6,6-Tetramethylpiperidin-1-oxy-4-ol ("4-OH-TEMPO" oder "TEMPOL"), 1-Oxyl-2,2,6,6-tetramethylpiperidin-4-on (TEMPON), 1-Oxyl-2,2,6,6-tetramethyl-4-carboxyl-piperidin (4-Carboxy-TEMPO), 1-Oxyl-2,2,5,5-tetramethylpyrrolidin, 4-methoxy-2,2,6,6-tetramethylpiperidin-N-Oxyl, 4-oxo-2,2,6,6-tetramethylpiperidin-N-Oxyl, 1-Oxyl-2,2,5,5-tetramethyl-3-carboxylpyrrolidin (3-Carboxy-PROXYL), 4-Benzoyloxy-2,2,6,6-tetramethylpiperidin-N-Oxid oder ferner Aluminium-N-nitrosophenylhydroxylamin oder Diethylhydroxylamin, das in Lösung oder trägergebunden vorliegen kann.

8. Verfahren nach einem der vorstehenden Ansprüche, wobei die Oxidation mit einem molaren Überschuss des Oxidationsmittels gegenüber 5-Hydroxymethylfurfural durchgeführt wird, vorzugsweise mit dem 4- oder mehr-, insbesondere dem 4,5 bis 7-fachen molaren Überschuss gegenüber dem 5-Hydroxymethylfurfural.

9. Verfahren nach einem der vorstehenden Ansprüche, **dadurch gekennzeichnet, dass** die Reaktion in Gegenwart von einem Metallhalogenid, insbesondere einem Alkali- oder Erdalkalimetallchlorid oder -bromid, stattfindet.

10. Verfahren nach einem der vorstehenden Ansprüche, **dadurch gekennzeichnet, dass** vor der dort beschriebenen Oxidation eine Disproportionierung zu 5-Hydroxymethylfuran-2-carbonsäure und 2,5-Dihydroxyxmethylfuran und, gewünschtenfalls nach Aufreinigung der 5-Hydroxymethylfuran-2-carbonsäure, deren weiterer Oxidation unter basischen Bedingungen wie in einem der vorstehenden Ansprüche beschrieben, stattfindet.

11. Verfahren nach einem der vorstehenden Ansprüche, wobei der Reaktionsmischung ein Lösungsmittel zugesetzt ist oder wird.

12. Verfahren nach einem der vorstehenden Ansprüche, **dadurch gekennzeichnet, dass** in einer weiteren parallelen oder nachfolgenden Reaktion die Furan-2,5-dicarbonsäure in einen Ester oder ein reaktives Furan-2,5-dicarbonsäurederivat, wie ein Halogenid, einen Anhydrid oder einen reaktiven Ester, umgewandelt wird.

13. Verfahren nach einem der Ansprüche 1 bis 12, **dadurch gekennzeichnet, dass** es als Continuous Flow-Verfahren durchgeführt wird.

14. Verfahren nach einem der Ansprüche 1 bis 12, **dadurch gekennzeichnet, dass** es als Batch-Verfahren durchgeführt wird.

15. Verfahren nach einem der vorstehenden Ansprüche, weiter umfassend die Herstellung des Ausgangsmaterials 5-Hydroxymethylfurfural durch Dehydratisierung von Hexosen oder hexosehaltigen Materialien aus Biomasse, wie Fructose.

16. Verfahren nach einem der vorstehenden Ansprüche, weiter umfassend die Kondensation des erhaltenen Furan-2,5-dicarbonsäureesters oder einem reaktionsfähigen Derivat davon mit einem Diol, wie Ethylenglykol, zur Herstellung von Polyesterharzen.

## Claims

1. Process for producing furan-2,5-dicarboxylic acid, including the oxidation of 5-hydroxymethylfurfural in a liquid reaction mixture in a one-pot process or in a continuous flow process; wherein a metal hypohalite salt is used as oxidizing agent and the 5-hydroxymethylfurfural starting material is a raw material obtained from biomass in the hydrothermal carbonization of hexoses or hexose-containing materials in the form of an aqueous solution or dispersion.

2. Process according to Claim 1, **characterized in that** the hexose-containing material is fructose.

3. Process according to Claim 1 or 2, **characterized in that** the reaction is carried out under phase transfer conditions.

4. Process according to any of the preceding claims, **characterized in that** the reaction under phase transfer conditions is carried out in the presence of a phase transfer catalyst, preferably in the presence of tetraalkylammonium sulfate such as tetrabutylammonium sulfate or dodecyltrimethylammonium sulfate or halides, such as bromide, chloride or sulfate.

5. Process according to any of the preceding claims, **characterized in that** the reaction is carried out under basic conditions, preferably adjusted by bases, for example basic alkali metal or alkaline earth metal salts, such as the corresponding carbonates or phosphates, or by alkali metal hydroxides, such as sodium or potassium hydroxide; wherein "under basic conditions" means in particular that the reaction takes place in a pH range of greater than 9, preferably from 9.5 to 13, for example from 9.8 to 12.5, wherein the pH is kept constant preferably by continuous addition of the appropriate base.

6. Process according to any of the preceding claims, wherein the oxidation is carried out with KOCl, LiOCl, NaOCl, Ca(OCl)₂ or a mixture of two or more of these hypochlorites as oxidizing agent.

7. Process according to any of the preceding claims, wherein the oxidation is carried out in the presence of a nitroxyl radical as catalyst, preferably of a tetramethylpiperidine oxide, such as 2,2,6,6-tetramethylpiperidine-1-oxyl (TEMPO), 2,2,6,6-tetramethylpiperidine-1-oxy-4-ol ("4-OH-TEMPO" or "TEMPOL"), 1-oxyl-2,2,6,6-tetramethylpiperidine-4-one (TEMPON), 1-oxyl-2,2,6,6-tetramethyl-4-carboxylpiperidine (4-Carboxy-TEMPO), 1-oxyl-2,2,5,5-tetramethylpyrrolidine, 4-methoxy-2,2,6,6-tetramethylpiperidine-N-oxyl, 4-oxo-2,2,6,6-tetramethylpiperidine-N-oxyl, 1-oxyl-2,2,5,5-tetramethyl-3-carboxylpyrrolidine, (3-Carboxy-PROXYL), 4-benzoyloxy-2,2,6,6-tetramethylpiperidine-N-oxide or also aluminium N-nitrosophenylhydroxylamine or diethylhydroxylamine, which may be present in solution or carrier-bound.

8. Process according to any of the preceding claims, wherein the oxidation is carried out with a molar excess of the oxidizing agent with respect to 5-hydroxymethylfurfural, preferably with a 4-fold or greater molar excess, especially a 4.5 to 7-fold molar excess with respect to 5-hydroxymethylfurfural.

9. Process according to any of the preceding claims, **characterized in that** the reaction takes place in the presence of a metal halide, particularly an alkali metal or alkaline earth metal chloride or bromide.

10. Process according to any of the preceding claims, **characterized in that**, prior to the oxidation described therein, disproportionation to 5-hydroxymethylfuran-2-carboxylic acid and 2,5-dihydroxymethylfuran takes place and, if desired after purification of the 5-hydroxymethylfuran-2-carboxylic acid, further oxidation thereof takes place under basic conditions as described in any of the preceding claims.

11. Process according to any of the preceding claims, wherein a solvent has been or is added to the reaction mixture.

12. Process according to any of the preceding claims, **characterized in that** in a further parallel or subsequent reaction, the furan-2,5-dicarboxylic acid is converted to an ester or a reactive furan-2,5-dicarboxylic acid derivative such as a halide, an anhydride or a reactive ester.

13. Process according to any of Claims 1 to 12, **characterized in that** said process is carried out as a continuous flow process.

14. Process according to any of Claims 1 to 12, **characterized in that** said process is carried out as a batch process.

15. Process according to any of the preceding claims, further comprising the production of the 5-hydroxymethylfurfural starting material by dehydrating hexoses or hexose-containing materials from biomass, such as fructose.

16. Process according to any of the preceding claims, further comprising condensation of the resulting furan-2,5-dicarboxylic acid ester or a reactive derivative thereof with a diol, such as ethylene glycol, to produce polyester resins.

## Revendications

1. Procédé de préparation d'acide 2,5-furanedicarboxylique, comprenant l'oxydation de 5-hydroxyméthylfurfural dans un mélange réactif liquide dans un procédé monotope ou dans un procédé à flux continu, selon lequel un sel d'hypohalogénite de métal est utilisé comme agent oxydant et la matière de départ 5-hydroxyméthylfurfural est une matière première produite sous la forme d'une solution aqueuse ou d'une dispersion à partir de biomasse lors de la carbonisation hydrothermale d'hexoses ou de matières contenant des hexoses.

2. Procédé selon la revendication 1, **caractérisé en ce que** la matière contenant des hexoses est du fructose.

3. Procédé selon la revendication 1 ou 2, **caractérisé en ce que** la réaction est effectuée dans des conditions de transfert de phase.

4. Procédé selon une des revendications précédentes, **caractérisé en ce que** la réaction dans des conditions de phase est réalisée en présence d'un catalyseur de transfert de phase, de préférence en présence de sulfate ou d'halogénures tels que du bromure, du chlorure ou du sulfate de tétraalkylammonium tel que du tétrabutylamminium ou du didécyltriméthylammonium.

5. Procédé selon une des revendications précédentes, **caractérisé en ce que** la réaction est exécutée dans des conditions basiques, de préférence ajustée par des bases, p. ex. des sels de métaux alcalins ou alcalino-terreux comme les carbonates ou les phosphates correspondants, ou par des hydroxydes de métaux alcalins comme de l'hydroxyde de sodium ou de potassium, "dans des conditions basiques" signifiant que la réaction a lieu sur une plage de pH supérieure à 9, de préférence de 9,5 à 13, p. ex. de 9,8 à 12,5, la valeur pH étant maintenue constante de préférence par l'ajout continu de la base correspondante.

6. Procédé selon une des revendications précédentes, selon lequel l'oxydation est réalisée avec du KOCl, du LiOCl, NaOCl, du Ca(OCl)₂ ou un mélange de deux ou davantage de ces hypochlorites comme agent oxydant.

7. Procédé selon une des revendications précédentes, selon lequel l'oxydation est réalisée en présence d'un radical nitroxyle comme catalyseur, de préférence d'un oxyde de tétraméthylpépiridine tel que du 2,2,6,6-tétraméthylpépiridine-1-oxyle (TEMPO), du 2,2,6,6-tétraméthylpépiridine-1-oxy-4-ol ("4-OH-TEMPO" ou "TEMPOL"), du 1-oxyle-2,2,6,6-tétraméthylpépiridine-1-on (TEMPON), du 1-oxyle-2,2,6,6-tétraméthyl-4-carboxyle-pipéridine (4-carboxy-TEMPO), du 1-oxyle-2,2,5,5-tétraméthylpyrrolidine, du 4-méthoxy-2,2,6,6-tétraméthylpipéridine-N-oxyle, du 4-oxo-2,2,6,6-tétraméthylpipéridine-N-oxyle, du 1-oxyle-2,2,5,5-tétraméthyle-3-carboxypyrrolidine (3-carboxy-PROXYL), du N-oxyde de 4-benzoyloxy-2,2,6,6-tétraméthylpripéridine ou encore de la N-nitrosophénylhydroxylamine d'aluminium ou de la diéthylhydroxylamine, qui peut se trouver en solution ou fixé sur un support.

8. Procédé selon une des revendications précédentes, selon lequel l'oxydation est réalisée avec excès molaire de l'agent oxydant par rapport au 5-hydroxyméthylfurfural, de préférence avec un excès molaire de 4 fois ou plus, en particulier de 4,5 à 7 fois par rapport au 5-hydroxyméthylfurfural.

9. Procédé selon une des revendications précédentes, **caractérisé en ce que** la réaction a lieu en présence d'un halogénure métallique, en particulier d'un chlorure ou d'un bromure de métal alcalin ou alcalino-terreux.

10. Procédé selon une des revendications précédentes, **caractérisé en ce qu'**avant l'oxydation décrite ici a lieu une dismutation en acide dicarboxylique de 5-hydroxyméthylfurane et n 2,5-dihydroxyméthylfurane et qu'éventuellement après purification de l'acide dicarboxylique de 5-hydroxyméthylfurane, son autre oxydation a lieu dans des conditions basiques comme dans une des revendications précédentes.

11. Procédé selon une des revendications précédentes, selon lequel un agent solvant est ajouté au mélange réactif ou est le mélange réactif.

12. Procédé selon une des revendications précédentes, **caractérisé en ce que**, dans une autre réaction parallèle ou suivante, l'acide 2,5-furanedicarboxylique est transformé en un ester ou un dérivé d'acide 2,5-furanedicarboxylique réactif tel qu'un halogénure, un anhydride ou un ester réactif

13. Procédé selon une des revendications 1 à 12, **caractérisé en ce qu'**il est réalisé comme un procédé à flux continu.

14. Procédé selon une des revendications 1 à 12, **caractérisé en ce qu'**il est réalisé comme un procédé discontinu.

15. Procédé selon une des revendications précédentes, comprenant en outre la préparation de la matière de départ 5-hydroxyméthylfurfural par déshydratation d'hexoses ou de matières contenant des hexoses issues de la biomasse tels que du fructose.

16. Procédé selon une des revendications précédentes, comprenant en outre la condensation de l'ester d'acide 2,5-furanedicarboxylique obtenu ou d'un dérivé réactif de celui-ci avec un diol tel que de l'éthylène glycol pour la préparation de résines polyesters.
